(19) Europäisches Patentamt
European Patent Office
Office européen des brevets

(11) **EP 2 031 387 B1**

(12) **EUROPEAN PATENT SPECIFICATION**

(45) Date of publication and mention
of the grant of the patent:
**27.01.2010 Bulletin 2010/04**

(51) Int Cl.:
***G01N 30/88*** *(2006.01)*   ***G01N 31/12*** *(2006.01)*
***B01D 59/44*** *(2006.01)*

(21) Application number: **07425511.8**

(22) Date of filing: **03.08.2007**

(54) **Reactor device for isotopic analyses**

Reaktorvorrichtung zur isotopischen Analyse

Dispositif réacteur pour analyses isotopiques

(84) Designated Contracting States:
**AT BE BG CH CY CZ DE DK EE ES FI FR GB GR
HU IE IS IT LI LT LU LV MC MT NL PL PT RO SE
SI SK TR**

(43) Date of publication of application:
**04.03.2009 Bulletin 2009/10**

(73) Proprietor: **Università degli Studi di Parma
43100 Parma (IT)**

(72) Inventors:
• **Boschetti, Tiziano
43100 Parma (IT)**

• **Iacumin, Paola
43100 Parma (IT)**

(74) Representative: **Lunati, Vittoriano et al
Lunati & Mazzoni S.r.l.
Via Carlo Pisacane, 36
20129 Milano (IT)**

(56) References cited:
**EP-A- 0 313 480     EP-A- 0 419 167
EP-A- 1 388 886     EP-A- 1 430 944
DE-C1- 19 816 348     US-A- 4 601 882**

Printed by Jouve, 75001 PARIS (FR)

**Description**

The present invention relates to a reactor device for isotopic analyses of the type specified in the preamble of claim 1 and as disclosed in DE-A-19816348.

There are currently known isotopic analyses, and in particular isotopic analysis of oxygen.

These analyses are performed to measure the abundance ratio R between the isotopes $^{18}O$ and $^{16}O$ of oxygen in a specific chemical compound, where R = $^{18}O/^{16}O$. The isotope composition of a compound is generally expressed with the $\delta$ (delta) notation, which expresses the difference between the isotope ratio R in the compound in relation to the same ratio in a standard of known composition:

$$\delta = [(Rsample - Rstandard) / Rstandard] \times 1000.$$

Determination of the parameter $\delta^{18}O$ is utilized for various purposes in different branches of sciences, for example in geology, in food sciences, in climatology and others.

For these purposes, isotopic analyses of organic and inorganic substances are performed.

In order to perform these analyses, in the 90s a very rapid and effective process based on carbothermic reduction was developed.

According to this principle and using specific reactors, the organic or inorganic compound to be analyzed is made to react at high temperature with carbon to produce carbon monoxide (CO) as the analysis gas.

By means of a continuous flow of inert gas (called carrier gas and usually realized by helium), the carbon monoxide thus obtained is sent from the reactor to a gas chromatography column to be separated from any other gaseous compounds and, following this, to known mass spectrometers for analysis of the abundance ratio and of the delta composition of the compound.

In particular, there have been developed reactors realized by a tubular reactor device inside which carbon monoxide formation takes place, connected at one end to a distributor device of samples to be analyzed (autosampler) and at the other end to an isotopes measurement apparatus including a mass spectrometer.

Said isotopic analysis method is also called online method as, due to the continuous flow of helium, the sample gas is obtained and isotopic analysis of the oxygen are performed in rapid succession and with minimum action by the operator.

One of the known reactor devices is, for example, realized by a tube made of ceramic refractory material, such as mullite (ceramic compound with variable proportions of alumina, $Al_2O_3$, and silica, $SiO_2$), connectable to the sample distributor and to the isotope measurement apparatus, disposed inside which is a further tube made of vitreous carbon filled for half the height thereof with a plurality of vitreous carbon elements. These latter elements have the dual function of supporting the portion of the sample to be analyzed in the area with the highest temperature and of providing, together with the inner tube, the carbon for the reaction.

The sample to be analyzed is then taken to high temperature, between 1300°C and 1450°C, so that the oxygen contained therein reacts totally with the vitreous carbon so as to be converted into carbon monoxide.

The carbon monoxide obtained in this manner is then pushed by the carrier flow of inert gas through the reactor device and, subsequently, to the gas chromatography column and to the isotopic composition measurement apparatus.

In particular, in said reactor device, the refractory tube has the function of isolating the vitreous carbon tube from the outside environment. In fact, vitreous carbon is unstable if exposed in air to the temperatures indicated, as it tends to oxidize starting from temperatures exceeding 450-500°C, producing carbon dioxide ($CO_2$) and carbon monoxide.

The aforesaid reactor has some drawbacks.

These drawbacks are linked to the fact that the external tube made of ceramic material is positioned very close to the internal tube made of vitreous carbon. In fact, at temperatures of over 1350°C and without introducing any sample, a background signal of carbon monoxide proportional to the temperature is generated. This unwanted carbon monoxide is linked to the well-known phenomenon of oxygen self-diffusion in the alumina and in the silicon and to the subsequent reaction of the oxygen, coming from the external tube, with the carbon supplied by the internal tube.

To solve the problem, reactor devices realized by a single tube made of silicon carbide (SiC) have been created. However, silicon carbide tends to be oxidized by carbon monoxide according to the reaction:

$$SiC(s) + 2CO(g) \rightarrow SiO_2(s) + 3C(s).$$

In fact, the chemical reaction indicated proceeds from left to right at the operating temperatures typical of isotopic analysis and at least up to 1515°C, temperature at which thermodynamic balance is obtained. The production of silica in the form of cristobalite removes oxygen from the carbon monoxide, resulting in isotopic fractionation and deterioration of the results not only of the sample being analyzed, but also of subsequent samples (cross contamination).

A further drawback of reactor devices currently marketed lies in the top limit of the analysis temperature. An electrical resistor made of silicon carbide (SiC) is generally used as heating element; this allows a normal operating temperature of 1400-1450°C to be obtained and temperatures of around 1550-1600°C can even be reached, but to the detriment of the duration thereof. This is due to the fact that at temperatures above 800°C and in contact with air, silicon carbide oxidizes and forms on the surface a protective film of silica ($SiO_2$) which, above 1300°C, causes the formation of cristobalite which protects the resistor from further oxidation. These silicon dioxide allotropes are poor conductors of electricity and thickening thereof causes an increase in the ohmic resistance of the heating element (phenomenon known as "aging"), which must be compensated by manually adjusting the voltage of a multiple output transformer. The use of the reactor at temperatures close to the limit for lengthy periods of time can cause breakage of the protective film, prematurely damaging the SiC heating element.

All this implies disadvantages linked to the limited life of the reactor device and to the relatively low temperature limits which preclude this method of analyzing the isotopic composition of the oxygen of many inorganic compounds, above all metal oxides and silicates, for which it is not possible to obtain an optimal yield of carbon monoxide.

Alternatively to the online methods, offline methods based on fluorination or other techniques are still used today for the aforesaid inorganic compounds.

However, these can cause health and safety hazards for operators, due to the use of reagents based on acid gases of halogens. In any case, they do not offer the rapidity and practicality of online methods.

In this situation the technical aim underlying the present invention is to devise a reactor device for isotopic analysis capable of substantially overcoming the aforesaid drawbacks.

Within said technical aim an important object of the invention is to obtain a reactor device for isotopic analysis capable of performing online analysis and providing a substantially correct measurement, not influenced by internal chemical reactions which alter the quantitative yield and/or composition of the carbon monoxide to be analyzed.

Another important object of the invention is to obtain a reactor device for isotopic analyses which has a high yield and a high duration in time.

The technical aim and the objects specified are attained by a reactor device for isotopic analyses as claimed in the appended Claim 1.

Preferred embodiments are highlighted in the dependent claims.

Other characteristics and advantages of the invention are further elucidated in the following detailed description of a preferred embodiment of the invention, given with reference to the attached drawings, wherein:

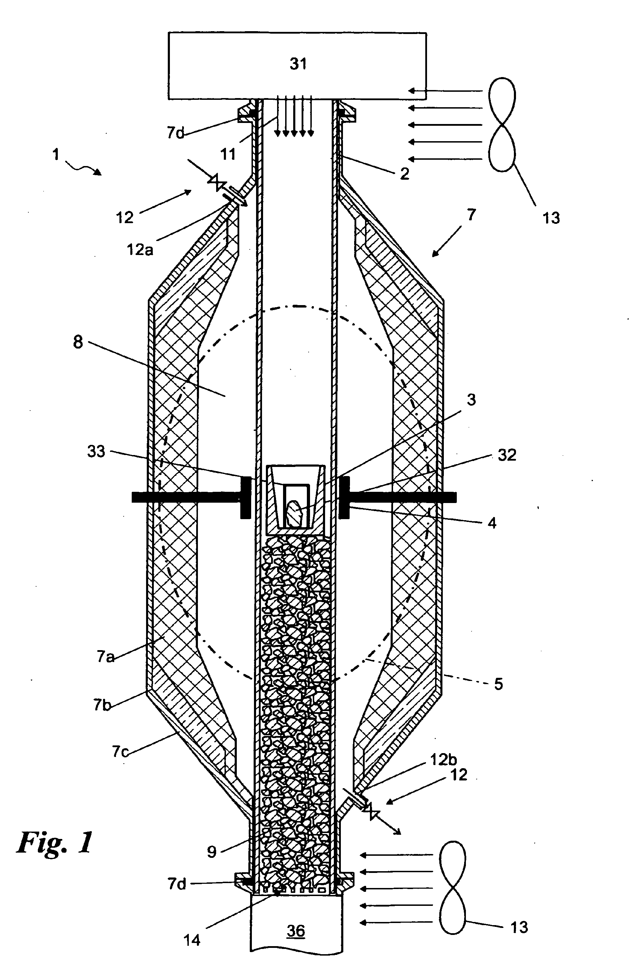

**Fig. 1** shows a reactor device for isotopic analyses according to the invention; and

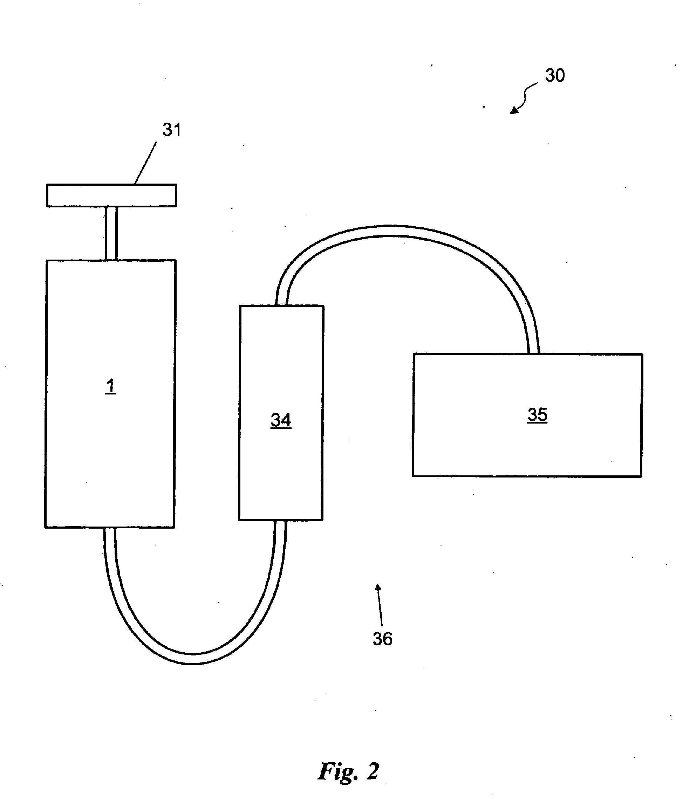

**Fig. 2** shows an apparatus for isotopic analyses in which the reactor device according to the invention is disposed.

With reference to the aforesaid Figures, the reactor device 1 according to the invention is indicated as a whole with the numeral **1.**

It is suitable to be disposed in an isotope measurement apparatus **30,** suitable to measure the isotopic ratio of oxygen $\delta^{18}O$.

The apparatus 30 comprises a sample distributor **31,** also called autosampler, of known type for solids or liquids and of standard dimensions suitable to allow samples of material **32** to be introduced into the apparatus 30. In particular, the samples of material 32 can be realized by organic or inorganic material in solid or liquid states.

The samples of solid or viscous liquid state are placed inside a manually reclosable capsule **33** of known type made of silver or, preferably, nickel with a high degree of purity.

The apparatus 30 also comprises the reactor device 1 according to the invention, suitable to bind the oxygen present in the sample 32 to the carbon to give rise to gaseous carbon monoxide according to the generic reaction:

$$MxOy\ (s,l,g) + yC(s) \rightarrow xM(s,l,g) + yCO(g)$$

where M is a generic element.

The apparatus 30 then comprises a gaseous molecule separator **34,** preferably realized by a gas chromatography column suitable to separate and select the various gas molecules coming from the reactor device 1 and to send the carbon monoxide to a mass spectrometer **35,** suitable to measure the isotopic composition of these particles of carbon monoxide and therefore the isotope ratio of the oxygen $\delta^{18}O$.

Finally, the apparatus comprises an electronic processor suitable to view the result of the measurement and to control the apparatus 30 and, preferably, also the device 1.

Said particle separator 34, mass spectrometer 35 and electronic processor are as a whole herein called isotope measurement system **36** and are realized by elements known per se and having standard characteristics and dimensions.

The reactor device 1 for isotopic analyses is, as specified, suitable to bind the oxygen present in this sample 32 to the carbon to give rise to gaseous carbon monoxide which is subsequently measured by the measurement system 36.

It comprises a tube **2** made of vitreous carbon preferably suitable to be connected directly to the sample distributor 31 and to the isotope measurement system 36.

For this purpose this vitreous carbon tube 2 has a diameter and thickness of standard dimensions and preferably between 0.2 mm and 5 mm respectively and a length preferably included in the measurement unit of tens of centimetres.

A crucible **3,** also made of vitreous carbon and suitable to contain the sample 32, is preferably disposed inside the vitreous carbon tube 2.

This crucible 3 is preferably realized by an external cylindrical shape, having a smaller diameter than the diameter of the tube 2, a closed lower base and an open upper base, suitable to allow samples 32 to be placed inside this crucible 3 and gaseous carbon monoxide to exit subsequent to completion of the chemical reaction described above. The inner walls of the crucible are inclined and convergent towards the base thereof, to allow a greater quantity of carbon available for the reaction and better cleaning.

The crucible 3 is preferably supported by specific supporting elements **9** appropriately realized by vitreous carbon chips suitable to fill the lower half of the tube 2.

The device 1 also comprises a heating element **4,** disposed outside the tube 2 and suitable to heat the crucible 3, and in particular the sample 32 present in the crucible, to temperatures at which the reaction of the carbon with the oxygen present in the sample 32 occurs to form the carbon monoxide to be measured.

For the purpose of facilitating said reaction, vitreous carbon powder, having a high specific reaction surface, or other catalysts such as anhydrous salts of halogens, can be placed in the crucible 3 or directly inside the capsule 33 containing the sample.

The heating element 4 is preferably realized by one or more electrical heating elements, or resistors, made of graphite, through which an electrical current flows and suitable to heat the crucible 3 by the Joule effect (also called ohmic heating) to temperatures exceeding 1325°C and preferably in the region of 2000°C so that a quantitatively complete reaction of conversion of the oxygen contained in the substance being analyzed into carbon monoxide occurs.

The heating element 4 produces a high temperature area **5,** in which the temperature is approximately 600°C higher, and in which the vitreous carbon is capable of reacting with compounds containing oxygen atoms.

A thermoregulator is then preferably provided in proximity of the heating element 4, preferably realized by a thermocouple with microprocessor, known per se and suitable to measure and maintain the desired temperature in said crucible 3.

Moreover, the device 1 includes first conveying means **11** of an inert gas, and appropriately of helium (He), inside the tube 2, preferably with inlet into the device 31 and connected to an inert gas bottle. The feed of inert gas into the reactor device is then regulated with a gauge or flow meter so that it is approximately 1 bar (pressure) or approximately 40-100 millilitres per minute (flow) during analysis.

This means 11 is suitable to maintain an inert atmosphere inside the tube 2 and to convey the gaseous carbon monoxide through this tube 2 to the isotope measurement system 36.

The reactor device 1 then comprises a casing **7** made of refractory material, defining a reaction chamber **8** which includes the heater 4 and at least part of the tube 2, preferably the central portion thereof.

The casing 7 is in direct contact with the tube 2 outside the high temperature area 5 so as to prevent this tube 2 from reacting with the casing 7; moreover, the walls of the casing 7 are in any case maintained at a lower temperature of approximately 320°C by heat dissipators, described below.

The casing 7 is also positioned at a distance from the central portion of the tube 2 greater than approximately 2 mm, so that, in the case in which it is made of ceramic materials or the like, no reactions occur between tube and casing 7.

Unlike other materials, the heating element 4, realized by graphite resistors, can be positioned in proximity of the vitreous carbon of which the tube 2 is made, as it is also substantially made of carbon.

The device 1 then comprises second conveying means **12** suitable to convey an inert gas, appropriately Helium (He), into the reaction chamber 8.

The means 12 is appropriately realized by two-way and gas-tight valves and is also connected to an inert gas bottle and preferably comprises an inlet duct **12a** and an outlet duct **12b** suitable to allow the delivery of inert gas from the reaction chamber 8.

In particular, the second conveying means 12 is suitable to produce inside the reaction chamber 8 an inert atmosphere, or in any case free of compounds containing oxygen.

For this purpose, the pressure of the inert gas inside the reaction chamber 8 is preferably greater than the external ambient, and in particular atmospheric (> 1 bar), pressure, so as to prevent non-inert gaseous elements, which could potentially react with the tube and the heating elements present in the high temperature area 5, from entering the chamber 8.

In order to prevent air from entering the reaction chamber 8, the upper part of the casing 7 is connected to the coupling with the sample distributor by seal elements **7d,** such as screw couplings with static O-rings made of plastic material (i.e. perfluorinated elastomer or another polymer resistant to high temperatures) or metal (i.e., stainless steel or another alloy resistant to high temperatures) suitable to maintain the gas-tight seal in the reaction chamber 8. An identical connection is utilized in the lower part.

The casing is also appropriately realized by an inner portion **7a,** which faces the crucible 3, made of carbon and in particular carbon felt, by an intermediate portion **7b,** which can also be omitted, made of ceramic refractory material, and finally by an outer surface **7c** made of stainless steel or another metal.

In particular, the intermediate portion 7b can have ceramic material containing oxygen in the form of refractory oxides or silicates, although this material must not face the reaction chamber 8 directly and can in any case be interposed with the zones 7a and 7c only outside the high temperature zone 5.

At the end portions of the tube 2, there are then disposed heat dissipators **13,** realized by fans or the like, suitable to cool said ends so as to position them outside the high temperature area 5 and so as to allow direct contact of the tube 2 with the parts of the casing 7a or 7c.

Finally, the device 1 comprises a supporting base **14** for the supporting elements 9, suitable to support said elements, to allow gas to pass through and inert to carbon monoxide (i.e. silver wool).

Operation of the reactor device 1, the structure of which is described above, is as follows.

To perform isotopic analysis of the oxygen contained in an organic or inorganic substance, a portion of specific dimensions and weight or volume is taken therefrom.

In the case of liquid substances, the specific volume can be injected manually or by means of a specific liquid sample distributor, also called liquid autosampler, with suitable gas-tight coupling with the tube 2.

In the case of viscous solids or liquids, the portion of substance forming the sample 32 is placed in a silver or nickel capsule 33.

In some cases the samples 32 are pulverized and mixed with vitreous carbon powder or other catalysts to obtain a faster reaction.

The capsule 33, containing the sample 32, is then inserted manually inside the sample distributor 31.

The same capsule 33 is then disposed by the distributor 31 inside the crucible 3, preferably by dropping it through the tube 2.

The heating element 4 must have been activated at the desired temperature prior to arrival in the capsule, as must the first and second conveying means 11 and 12, and the heat dissipators 13.

The metal constituting the capsule 33 is then melted and collected in the crucible, allowing the sample 32 to interact at high temperatures.

In particular, the capsule 33 does not interact with the sample 32 as it is made of a noble metal or in any case one that does not react with the sample 32.

The sample 32 instead reacts with the vitreous carbon of the crucible 3 or of the carbon powder contained therein.

In particular, the reaction indicated previously occurs and gaseous carbon monoxide forms, which exits from the upper base of the crucible 3 and is conveyed by the flow of inert gas through the supporting elements 9 in the tube 2 to reach the isotope measurement system 36.

During this passage, the carbon monoxide does not bind with potentially reactive elements; in fact, it comes into contact, in the high temperature area 5, exclusively with elements made of vitreous carbon or non-reactant material.

Likewise, the external and internal surface of the tube 2 does not react with any element due to the presence of an inert atmosphere and to the appropriate distance of the casing 7 in the area 5.

The invention achieves important advantages.

In fact, due to the lack of reactivity of the carbon monoxide and of the tube 2 with other elements, isotopic analyses of the oxygen are precise and correct.

It is therefore possible to perform rapid online isotopic analyses with high precision.

Moreover, having a tube 2 of standard dimensions connectable directly to the distributor 31 and to the measurement system 36, the device 1 can be utilized with systems of known type.

Moreover, due to a higher reaction temperature, this device 1 allows isotopic analysis of the oxygen also in refractory compounds.

The device 1 also has a high duration in time, as the tube 2 does not react with external elements. Besides reducing the costs of expendable material related to the presence of two tubes, the presence of a single tube increases the inner reaction volume of the tube 2, thus allowing a higher number of samples to be analyzed.

Finally, the capsule 33 made of nickel, has the dual advantage of being autocatalytic in the presence of carbon, due to the known pyrophoric properties of nickel at high temperatures and in the presence of carbon. Moreover, nickel inhibits the production of metal gases at peak temperatures, thanks to the higher boiling point with respect to silver.

## Claims

1. Reactor device (1) for isotopic analyses of a sample (32) suitable to be connected to a distributor (31) of samples (32) and to an isotope measurement system (36) suitable to measure the isotopic composition of carbon monoxide, comprising: a tube (2) made of vitreous carbon, including a crucible (3) made of vitreous carbon suitable to contain said sample (32), a heating element (4), disposed outside said tube (2), suitable to take said crucible (3) to reaction temperatures of the carbon with the oxygen present in said sample (32) so as to form carbon monoxide, said heating element (4) defining a high temperature area (5) in which the vitreous carbon is suitable to react with the oxygen, and first conveying means (11) suitable to convey a flow of inert gas into said tube (2) to convey said carbon monoxide to said isotope measurement system (36) through said tube (2) and to maintain an inert atmosphere inside said tube (2), **characterized in that** it comprises a casing (7) made of refractory material defining a reaction chamber (8) which comprises said heating element (4) and at least part of said tube (2), said casing (7) being in contact with this tube (2) outside said high temperature area (5), and second conveying means (12) suitable to convey an inert gas into said reaction chamber (8).

2. Reactor device according to claim 1, wherein said heating element (4) is constituted by graphite resistors.

3. Reactor device according to claim 2, wherein said graphite resistors are suitable to heat said crucible (3) by the Joule effect.

4. Reactor device according to one or more of the preceding claims, wherein said tube (2) is directly connectable to said sample distributor (31) and to said isotope measurement system (36).

5. Reactor device according to one or more of the preceding claims, wherein said casing (7) comprises an inner portion (7a) made of carbon, facing said crucible (3).

6. Reactor device according to claim 5, wherein said inner portion (7a) is made of carbon felt.

7. Reactor device according to one or more of the preceding claims, wherein said conveying means (12) is suitable to produce inside said reaction chamber (8) an inert atmosphere at a higher pressure than the external pressure.

8. Reactor device according to one or more of the preceding claims, wherein a thermoregulator is provided in proximity of said heating element (4), realized by a thermocouple with microprocessor and suitable to measure and maintain the temperature in said crucible (3).

9. Reactor device according to one or more of the preceding claims, comprising a capsule (33) made of nickel suitable to contain said sample (32).

10. Isotope measurement apparatus (30), suitable, to measure the isotopic ratio $\delta^{18}O$, comprising: a sample distributor (31), suitable to allow samples of material (32) to be introduced into said apparatus (30), a reactor device (1) according to one or more of the preceding claims, a gaseous molecule separator (34), suitable to separate and select the gases coming from said reactor device (1) and to select carbon monoxide gas to be sent to a mass spectrometer (35), suitable to measure the isotopic composition of said carbon monoxide gas.

## Patentansprüche

1. Reaktorvorrichtung (1) für Isotopenanalysen einer Probe (32), die an einen Verteiler (31) von Proben (32) und an ein zur Bestimmung der Kohlenmonoxydisotopenzusammensetzung geeignetes Isotopenmesssystem (36) verbunden werden kann, bestehend aus: einem Rohr (2) aus Glaskohlenstoff, umfassend einen Tiegel (3) aus Glaskohlenstoff, der genannte Probe (32) beinhaltet, einer Heizvorrichtung (4), die das Äußere des genannten Rohres (2) umgibt und den genannten Tiegel (3) auf Temperaturen bringt, in denen der Kohlenstoff mit dem in genannter Probe (32) anwesenden Sauerstoff reagiert, sodass sich Kohlenstoffmonoxyd bildet, wobei genannte Heizvorrichtung (4) eine Hochtemperaturzone (5) bestimmt, an deren Höhe der Glaskohlenstoff in der Lage ist, mit dem Sauerstoff zu reagieren, und ersten Fördermittel (11), die in der Lage sind, einen Inertgasfluss in das Innere des genannten Rohres (2) zu leiten, um genannten Monoxydkohlenstoff zu genanntem Isotopenmeßsystem (36) über genanntes Rohr (2) zu befördern und im Inneren des genannten Rohres (2) eine Schutzatmosphäre zu erhalten, **dadurch gekennzeichnet, dass** sie eine Umhüllung (7) aus hitzebeständigem Material umfasst, die eine Reaktionskammer (8) bildet, die genannte Heizvorrichtung (4) und mindestens Teil des genannten Rohres (2) umfasst, wobei genannte Umhüllung am gleichen Rohr außerhalb der genannten Hochtemperaturzone (5) anliegt, und zweite Fördermittel (12), die in der Lage sind, ein Inertgas in das Innere der genannten Reaktionskammer (8) zu leiten.

2. Reaktorvorrichtung nach Anspruch 1, in der die genannte Heizvorrichtung (4) aus Kohlewiderständen besteht.

3. Reaktorvorrichtung nach Anspruch 2, in der genannte Kohlewiderstände den genannten Tiegel (3) durch Joule-Effekt erhitzen.

4. Reaktorvorrichtung nach einem oder mehreren vorstehenden Ansprüchen, in der genanntes Rohr (2) an genannten Probenverteiler (31) und an genanntes Isotopenmesssystem (36) direkt verbunden werden kann.

5. Reaktorvorrichtung nach einem oder mehreren vorstehenden Ansprüchen, in der genannte Umhüllung (7) einen Innenteil (7a) aus Kohlenstoff umfasst, der zu genanntem Tiegel (3) gerichtet ist.

6. Reaktorvorrichtung nach Anspruch 5, in der genannter Innenteil (7a) aus Kohlenstoff-Filz ist.

7. Reaktorvorrichtung nach einem oder mehreren vorstehenden Ansprüchen, in der genannte Fördermittel (12) in der Lage sind, im Inneren der genannten Reaktionskammer (8) eine Schutzatmosphäre mit höherem Druck als der Außendruck zu erzeugen.

8. Reaktorvorrichtung nach einem oder mehreren vorstehenden Ansprüchen, in der in der Nähe der genannten Heizvorrichtung (4) ein Wärmeregler vorgesehen ist, bestehend aus einem Thermoelement mit Mikroprozessor und in der Lage, die Temperatur in der Höhe des genannten Tiegels (3) zu messen und zu erhalten.

9. Reaktorvorrichtung nach einem oder mehreren vorstehenden Ansprüchen, umfassend eine Kapsel (33) aus Nickel zum Enthalten der genannten Probe (32).

10. Isotopenmessvorrichtung (30) zum Messen des Isotopenverhältnisses $\delta^{18}$O, bestehend aus: einem Probenverteiler (31) zum Einführen der Materialproben (32) in genannte Vorrichtung (30), einer Reaktorvorrichtung (1) nach einem oder mehreren vorstehenden Ansprüchen, einem Abscheider für gasförmige Moleküle (34) zum Trennen und Wählen der aus genannter Reaktorvorrichtung (1) kommenden Gase und zum Wählen von Kohlenstoffmonoxydgas, das an ein Massenspektrometer (35) zum Messen der Isotopenzusammensetzung des genannten Kohlenstoffmonoxydgases geleitet wird.

## Revendications

1. Dispositif réacteur (1) pour les analyses isotopiques d'un échantillon (32) apte à être relié à un distributeur (31) d'échantillons (32) et à un système de mesurage des isotopes (36) apte à mesurer la composition isotopique de l'oxyde de carbone, comprenant: un tube (2) réalisé en carbone vitreux, comprenant un creuset (3) réalisé en carbone vitreux destiné a contenir ledit échantillon (32), un élément chauffant (4), disposé à l'extérieur dudit tube (2), destiné à amener ledit creuset (3) à des températures de réaction du carbone avec l'oxygène présent dans ledit échantillon (32) de manière à former de l'oxyde de carbone, ledit élément chauffant (4) définissant une aire (5) à température élevée à laquelle le carbone vitreux est apte à réagir avec l'oxygène, et des premiers moyens de transport (11) aptes à transporter un écoulement de gaz inerte dans ledit tube (2) pour transporter ledit oxyde de carbone audit système de mesurage des isotopes (36) à travers ledit tube (2) et à maintenir une atmosphère inerte à l'intérieur dudit tube (2), **caractérisé en ce qu'**il comprend une enveloppe (7) en matériau réfractaire définissant une chambre de réaction (8) comprenant ledit élément chauffant (4) et au moins une partie dudit tube (2), ladite enveloppe (7) étant en contact avec ce tube (2) au-dehors de ladite aire (5) à température élevée, et des deuxièmes moyens de transport (12) aptes à transporter un gaz inerte dans ladite chambre de réaction (8).

2. Dispositif réacteur selon la revendication 1, dans lequel ledit élément chauffant (4) se compose de résistances de graphite.

3. Dispositif réacteur selon la revendication 2, dans lequel lesdites résistances de graphite sont aptes à chauffer ledit creuset (3) par effet Joule.

**4.** Dispositif réacteur selon une ou plusieurs des revendications précédentes, dans lequel ledit tube (2) peut être relié directement audit distributeur d'échantillons (31) et audit système de mesurage des isotopes (36).

**5.** Dispositif réacteur selon une ou plusieurs des revendications précédentes, dans lequel ladite enveloppe (7) comprend une portion intérieure (7a) en carbone tournée vers ledit creuset (3).

**6.** Dispositif réacteur selon la revendication 5, dans lequel ladite portion intérieure (7a) est en feutre de carbone.

**7.** Dispositif réacteur selon une ou plusieurs des revendications précédentes, dans lequel lesdits moyens de transport (12) sont aptes à produire à l'intérieur de ladite chambre de réaction (8) une atmosphère inerte à une pression plus élevée que la pression extérieure.

**8.** Dispositif réacteur selon une ou plusieurs des revendications précédentes, dans lequel on prévoit un thermorégulateur à proximité dudit élément chauffant (4), réalisé par un couple thermoélectrique avec microprocesseur et apte à mesurer et maintenir la température dans ledit creuset (3).

**9.** Dispositif réacteur selon une ou plusieurs des revendications précédentes, comprenant une capsule (33) fabriquée en nickel apte à contenir ledit échantillon (32).

**10.** Appareil de mesurage d'isotopes (30), apte à mesurer le rapport isotopique $\delta^{18}O$, comprenant: un distributeur d'échantillons (31), apte à permettre l'introduction d'échantillons de matériau (32) dans ledit appareil (30), un dispositif réacteur (1) selon une ou plusieurs des revendications précédentes, un séparateur de molécules gazeuses (34) apte a séparer et sélectionner les gaz en provenance dudit dispositif réacteur (1) et à sélectionner le gaz d'oxyde de carbone à envoyer à un spectromètre de masse (35), apte à mesurer la composition isotopique dudit gaz d'oxyde de carbone.

**Fig. 1**

*Fig. 2*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 19816348 A